# DEMANDE DE BREVET EUROPEEN

(11) **EP 1 649 894 A2**
(43) Date de publication de la demande: **26.04.2006**
(21) Numéro de dépôt: 05292141.8
(22) Date de dépôt: 13.10.2005
(51) Int. Cl.: A61Q 5/00, A61Q 5/12, A61K 8/40, A61K 8/49, A61K 8/55

(54) **Composition comprenant des monomères électrophiles et des sels organiques particuliers, et son utilisation pour le traitement cosmétique des matières kératiniques**

(30) Priorité: 13.10.2004 FR 0410810
(71) Demandeur: L'OREAL, 75008 Paris (FR)
(72) Inventeur: Samain, Henri, 91570 Bièvres (FR); Giroud, Franck, 92110 Clichy (FR)
(74) Mandataire: Casalonga, Axel

(57) **Abrégé**

La présente invention concerne une composition comprenant dans un milieu cosmétiquement acceptable, au moins un monomère électrophile et au moins un sel organique non polymérique présentant un point de fusion inférieur à 60 °C, et son utilisation pour le traitement cosmétique des matières kératiniques.

Elle concerne aussi un procédé de traitement cosmétique des matières kératiniques, en particulier des fibres kératiniques telles que les cheveux, mettant en oeuvre ladite composition.

Elle concerne également un procédé de traitement cosmétique des matières kératiniques comprenant une étape d'application dudit sel organique non polymérique, et une autre étape d'application d'au moins un monomère électrophile.

## Description

La présente invention est relative à une composition comprenant au moins un monomère électrophile et au moins sel organique particulier, à son utilisation pour le traitement cosmétique des matières kératiniques et à un procédé de traitement cosmétique mettant en oeuvre une telle composition.

Dans le domaine de la cosmétique, on cherche à modifier les propriétés superficielles des matières kératiniques telles que les fibres kératiniques comme les cheveux, par exemple pour apporter aux cheveux un effet conditionnant comme la douceur, ou de la brillance. Pour ce faire, on utilise généralement des compositions cosmétiques à base d'agents de conditionnement tels que des silicones ou des polymères ayant une forte affinité pour les matières kératiniques, et notamment pour les cheveux.

Cependant, ces agents de conditionnement ont tendance à s'éliminer au cours de lavage avec des shampoings, rendant nécessaire le renouvellement des applications des compositions sur les cheveux.

Pour accroître la rémanence de dépôt de polymères, il peut être envisagé d'effectuer une polymérisation radicalaire de certains monomères directement sur les cheveux. Toutefois, on constate une forte dégradation des fibres capillaires liées probablement aux amorceurs de polymérisation et les cheveux ainsi traités sont difficilement démêlables.

La Demanderesse a trouvé de manière surprenante qu'en utilisant l'association d'au moins un sel organique non polymérique particulier à au moins un monomère électrophile tel que décrit ci-dessous, il était possible d'obtenir un conditionnement et une brillance améliorés des cheveux, et ce de manière durable.

En effet, une composition comprenant une telle association permet de maintenir la douceur et la brillance apportées à la chevelure par ladite composition, et ceci sans ré-application, même après plusieurs lavages de la chevelure.

L'application d'une composition comprenant une telle association conduit à la formation in situ d'un revêtement lubrifiant et brillant rémanent, notamment aux shampoings.

En outre, les cheveux restent de manière surprenante parfaitement individualisés et peuvent être coiffés sans problème.

L'invention a donc pour objet une composition comprenant dans un milieu cosmétiquement acceptable, au moins un monomère électrophile et au moins un sel organique non polymérique présentant un point de fusion inférieur à 60 °C.

Un autre objet de la présente invention consiste en l'utilisation de ladite composition pour le traitement cosmétique des matières kératiniques, et plus particulièrement des fibres kératiniques comme les cheveux.

L'invention a encore pour objet un procédé de traitement cosmétique des matières kératiniques, et plus particulièrement des fibres kératiniques comme les cheveux, mettant en oeuvre ladite composition.

D'autres objets, caractéristiques, aspects et avantages de l'invention apparaîtront encore plus clairement à la lecture de la description et des exemples qui suivent.

Selon l'invention, la composition comprend dans un milieu cosmétiquement acceptable, au moins un monomère électrophile et au moins un sel organique non polymérique présentant un point de fusion inférieur à 60 °C.

Les sels organiques utilisables dans la présente invention font partie d'une classe générale de composés bien connus sous le nom de "Room Temperature Ionic Liquid ou RTIL". Ces RTIL présentent généralement un point de fusion inférieur à 100 °C et restent liquides jusqu'à une température de 300 °C environ.

Ces RTIL sont notamment décrits dans " Eyes on lonic Liquids, Chemical and Engineering News", May 15, 2000, Vol. 78, 20, pages 37-50 et dans "New Horizons For lonic Liquids, Chemical and Engineering News", January 1, 2001, Vol. 79, 1, pages 21-25.

Le point de fusion est mesuré par analyse calorimétrique différentielle (DSC) avec une vitesse de montée en température de 10°C/min. Le point de fusion est alors la température correspondant au sommet du pic endotherme de fusion obtenu lors de la mesure.

Le point de fusion du sel organique est de préférence inférieur à 20°C, mieux encore inférieur à 0°C et encore plus préférentiellement inférieur à -30 °C.

Les sels organiques utilisés dans le cadre de l'invention possèdent en outre un excellent pouvoir de solvatation et une excellente conductivité électrique. Ils sont également non-volatiles et ininflammables.

Un autre avantage de ces sels organiques réside dans le fait qu'ils sont facilement recyclables et font partie des solvants dits "verts".

Associés à au moins un monomère électrophile tel que décrit ci-dessous, ils permettent d'obtenir un revêtement lubrifiant et brillant, rémanent.

De préférence, les sels organiques peuvent être choisis parmi les sels d'imidazolium, de pyrazolium, de pyridinium, de pyrimidinium, de tétra-alkylphosphonium et de tétra-alkylammonium, de point de fusion inférieur à 60 °C, et leurs mélanges.

A titre d'exemples de sels d'imidazolium, on peut notamment citer ceux de formule (I) : 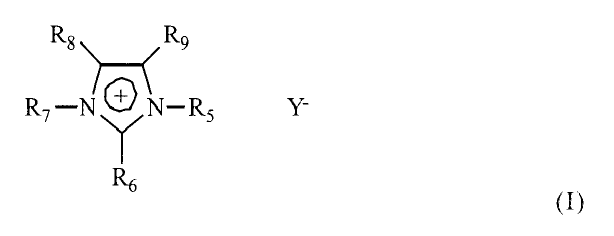 dans laquelle :
R₅ et R₇, identiques ou différents, représentent chacun un groupe alkyle comportant de 1 à 12 atomes de carbone, de préférence de 1 à 5 atomes de carbone, et mieux encore de 1 à 4 atomes de carbone,
R₆, R₈ et R₉, identiques ou différents, représentent chacun un atome d'hydrogène ou un groupe alkyle comportant de 1 à 4 atomes de carbone et de préférence de 1 à 3 atomes de carbone, et
Y⁻ représente un anion.

Les groupes alkyle peuvent être linéaires ou ramifiés. On peut notamment citer, par exemple, les groupes méthyle, éthyle, n-propyle, isopropyle, n-butyle, tert-butyle, pentyle, hexyle, octyle, nonyle, décyle et dodécyle.

On utilise de préférence dans la composition de l'invention, les sels d'(alkyl en C₁₋₁₀)-méthylimidazolium, et plus spécifiquement les sels de 1-butyl-3-méthylimidazolium ou de 1-éthyl-3-méthylimidazolium.

Comme sels de pyrazolium utilisables dans la présente invention, on peut notamment citer ceux de formule (II) : 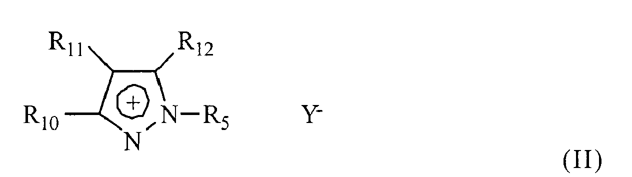 dans laquelle :
R₅ et Y⁻ a la même signification que ci-dessus, et
R₁₀, R₁₁ et R₁₂, identiques ou différents, représentent chacun un atome d'hydrogène ou un groupe alkyle comportant de 1 à 12 atomes de carbone et de préférence de 1 à 4 atomes de carbone.

Comme exemples de sels de pyridinium pouvant être utilisés dans la composition de l'invention, on peut notamment citer ceux de formule (III) : 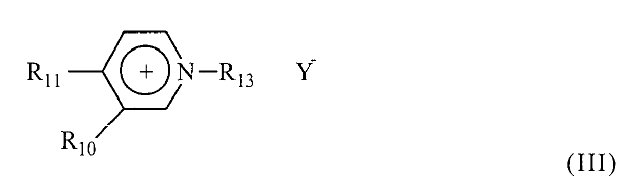 dans laquelle R₁₀, R₁₁ et Y⁻ sont tels que définis ci-dessus, et R₁₃ représente un groupe alkyle comportant de 1 à 12 atomes de carbone et de préférence de 1 à 4 atomes de carbone ;
et plus particulièrement les sels de N-butylpyrdinium.

Comme sels de pyrimidinium utilisables dans la présente invention, on peut notamment citer ceux de formules (IV) et (IV') : 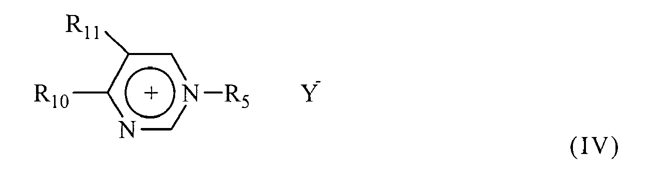 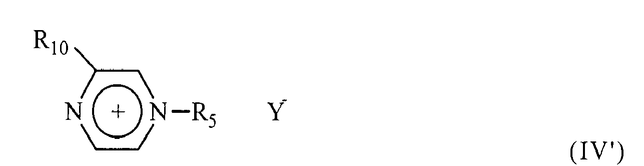 dans lesquelles R₅, R₁₀, R₁₁, et Y⁻ sont tels que définis ci-dessus.

D'autres sels organiques existent sous la forme de cations non hétérocycliques tels que les sels de tétra-alkylphosphonium et de tétra-alkylammonium de point de fusion inférieur à 60 °C.

Comme sels de tétra-alkylphosphonium ou de tétra-alkylammonium utilisables dans la présente invention, on peut notamment citer ceux de formule (V) ou (VI) : 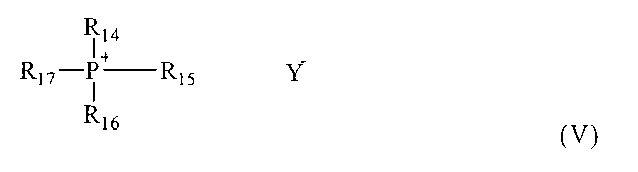 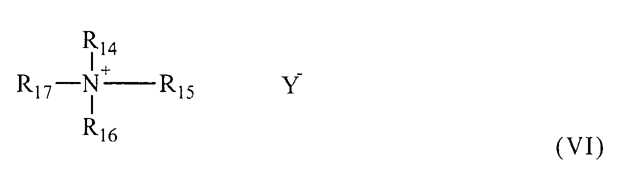 dans lesquelles R₁₄, R₁₅, R₁₆ et R₁₇, identiques ou différents, représentent un groupe alkyle comportant de 1 à 18 atomes de carbone et de préférence de 1 à 14 atomes de carbone, et Y⁻ représente un anion.

L'anion présent dans le sel organique, représenté par Y⁻, peut être tout anion bien connu dans la technique, et en particulier un ion chlorure (Cl⁻), un ion bromure (Br⁻), un ion tétrachloroaluminate (AlCl₄⁻), un ion tétrachloronickel (NiCl₄⁻), un ion perchlorate (ClO₄⁻), , un ion nitrate (NO₃⁻), un ion nitrite (NO₂⁻), un ion sulfate (SO₄²⁻), un ion méthylsulfate (CH₃SO₄⁻), un ion tétrafluoroborate (BF₄⁻), un ion hexafluorophosphate (PF₆⁻), un ion hexafluoroantimonate (SbF₆⁻), un ion triflate [TfO] (CF₃SO₂⁻) un ion nonaflate [NfO] (CF₃(CF₂)₃SO₂⁻), un ion [Tf₂N] (CF₃SO₂)₂N⁻), un ion trifluoroacétate [TA] (CF₃CO₂⁻), un ion heptafluorobutanoate [HB] (CF₃(CF₂)₃CO₂⁻), un ion acétate (CH₃CO₂⁻), ou un ion trifluorométhanesulfonate (CF₃SO₃⁻).

Parmi les anions cités ci-dessus, on préfère tout particulièrement les ions chlorure, bromure, sulfate, acétate, tétrafluoroborate, hexafluorophosphate, triflate, nonaflate, [Tf₂N] (CF₃SO₂)₂N⁻) et heptafluorobutanoate.

D'autres classes de sels organiques utilisables dans les compositions de l'invention sont citées dans la demande WO 2004/035018.

A titre d'exemples de sel organique utilisés dans le cadre de la présente invention, on peut notamment citer les sels organiques suivants :
le chlorure de 1-éthyl-3-méthylimidazolium,
le bromure de 1-éthyl-3-méthylimidazolium,
le chlorure de 1-butyl-3-méthylimidazolium,
le chlorure de 1-hexyl-3-méthylimidazolium,
le chlorure de 1-méthyl-3-octylimidazolium,
le chlorure de 1-décyl-3-méthylimidazolium,
le bromure de 1-décyl-3-méthylimidazolium,
le chlorure de 1-dodécyl-3-méthylimidazolium,
le chlorure de 1-méthyl-3-tétradécylimidazolium,
le chlorure de 4-méthyl-N-butyl-pyridinium,
le chlorure de 3-méthyl-N-butylpyridinium,
le chlorure de 4-méthyl-N-hexylpyridinium,
le tétrafluoroborate de 1-éthyl-3-méthylimidazolium,
le tétrafluoroborate de 1-butyl-3-méthylimidazolium,
le tétrafluoroborate de 1-pentyl-3-méthylimidazolium,
le tétrafluoroborate de 1-hexyl-3-méthylimidazolium,
le tétrafluoroborate de 1-heptyl-3-méthylimidazolium,
le tétrafluoroborate de 1-octyl-3-méthylimidazolium,
le tétrafluoroborate de 1-nonyl-3-méthylimidazolium,
le tétrafluoroborate de 1-décyl-3-méthylimidazolium,
le tétrafluoroborate de 4-méthyl-N-butylpyridinium,
le tétrafluoroborate de 1-hexyl-3-éthylimidazolium,
l'hexafluorophosphate de 1-éthyl-3-méthylimidazolium,
l'hexafluorophosphate de 1-butyl-3-méthylimidazolium,
l'hexafluorophosphate de 1-pentyl-3-méthylimidazolium,
l'hexafluorophosphate de 1-hexyl-3-méthylimidazolium,
l'hexafluorophosphate de 1-heptyl-3-méthylimidazolium,
l'hexafluorophosphate de 1-octyl-3-méthylimidazolium,
l'hexafluorophosphate de 1-nonyl-3-méthylimidazolium,
l'hexafluorophosphate de 1-décyl-3-méthylimidazolium,
le méthylsulfate de 1,3-diméthylimidazolium,
le méthylsulfate de 1-méthyl-3-butylimidazolium,
le nitrate de 1-éthyl-3-méthylimidazolium,
le nitrite de 1-éthyl-3-méthylimidazolium,
l'acétate de 1-éthyl-3-méthylimidazolium,
le sulfate de 1-éthyl-3-méthylimidazolium,
les triflates de 1-éthyl-3méthylimidazolium,
les nonaflates de 1-éthyl-3-méthylimidazolium,
le bis(trifyl)amide de 1-éthyl-3-méthylimidazolium,
le bromure de 1-butylpyridinium,
le trifluorométhanesulfonate de 1-butylpyrimidinium,
le trifluorométhanesulfonate de 1-hexylpyrimidinium,
le trifluoroacétate de 1-éthyl-3-méthylimidazolium,
le chlorure de trihexyl-tétradécyl-phosphonium,
le chlorure de tributyl-tétradécyl-phosphonium,
le tétrafluoroborate de 1-éthyl-2-méthylpyrazolium,
le tétrafluoroborate de 1-méthyl-3-butylpyrimidinium, et
le sulfate de trioctylammonium.

Il est possible de modifier la chimie des sels organiques telle qu'elle a été décrite précédemment pour faire varier leur solubilité. Pour conserver le caractère fusible à basse température de ces sels, il est préférable de modifier plus spécifiquement les chaînes alkyle constituant en partie les sels organiques. Dans ce cadre, on peut citer, à titre d'exemple, l'éthérification de la chaîne alkyle des sels de la famille des 1-alkyl-3-méthylimidazolium qui permet d'obtenir les composés de formules suivantes :

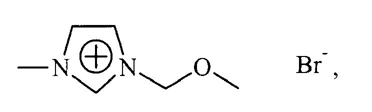

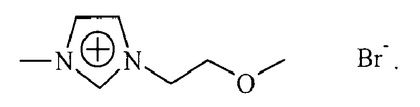

Une telle modification chimique donne ou renforce pour le sel organique le caractère hydrosoluble, sans perdre le caractère fusible à basse température du sel. Une telle modification chimique peut également permettre de faire gélifier les sels organiques par association avec des sucres amphiphiles et d'obtenir ce que l'on appelle des ionogels. De telles propriétés sont décrites dans l'article *"Spontaneous Self assembly of glycolipid bilayer Membranes in Sugarphilic Ionic Liquids and formation of Ionogels"* publié par N. Kimizuka et T Nakashima (Langmuir 17, 6759-6761, (2001)).

Le sel organique ou le mélange de sels organiques tels que décrits ci-dessus, est présent dans les compositions cosmétiques de l'invention, en une concentration comprise entre 0,001% et 95% en poids, de préférence entre 0,1% et 50% en poids, et mieux encore entre 0,2 % et 20 % en poids, par rapport au poids total de la composition.

Le sel organique ou le mélange de sels organiques peut se présenter tel quel ou en solution ou sous forme d'une émulsion. Le sel organique ou le mélange de sels organiques peut être également préalablement microencapsulé avant incorporation dans la composition cosmétique.

Par monomère électrophile, on entend un monomère capable de polymériser par polymérisation anionique en présence d'un agent nucléophile tel que par exemple, les ions hydroxyle (OH⁻) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
(i) les dérivés benzylidene malononitrile (A), le 2-(4-chloro-benzylidene)-malononitrile (A1), le 2-cyano-3-phényl acrylate d'éthyle (B), le 2-cyano-3-(4-chloro-phényl) acrylate d'éthyle (B1) tels que décrits dans Sayyah, J. Polymer Research, 2000, p97 : 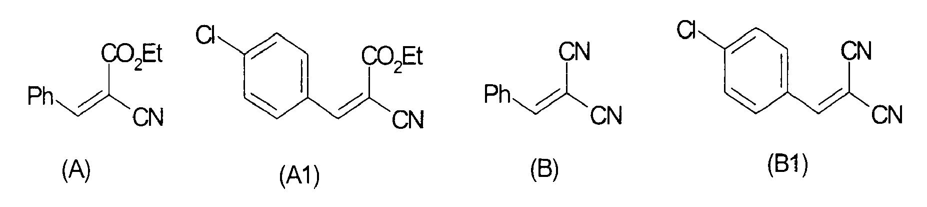
(ii) les dérivés de méthylidenemalonates comme :
   - le 2-méthylene-malonate de diéthyle (C) tel que décrit par Hopff, Makromoleculare Chemie, 1961, p95, par De Keyser, J. Pharm. Sci, 1991, p67 et par Klemarczyk, Polymer, 1998, p173: 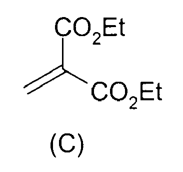
   - le 2-éthoxycarbonylméthyleneoxycarbonyl acrylate d'éthyle (D) tel que décrit par Breton, Biomaterials, 1998, p271 et Couvreur, Pharmaceutical Research, 1994, p1270 : 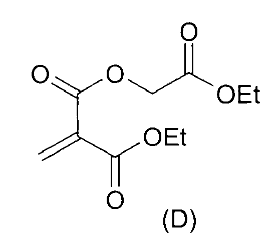
(iii) les dérivés itaconate et itaconimide comme :
   - l'itaconate de diméthyle (E) tel que décrit par Bachrach, European Polymer Journal, 1976, p563 : 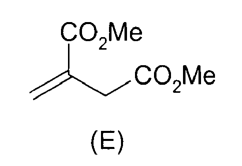
   - le N-butyl itaconimide (F), le N-(4-tolyl) itaconimide (G), le N-(2-ethylphenyl) itaconimide (H), le N-(2,6-diethylphenyl) itaconimide (I) tel que décrits par Wanatabe, J.Polymer Science : Part A :Polymer chemistry, 1994, p2073 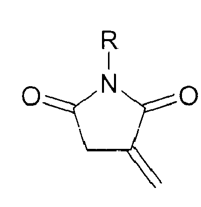
   R= Bu (F), 4-tolyl (G), 2-ehylphenyl (H), 2,6-diethyphenyl (I)
(iv) les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-(tert-butylsulfonyl)acrylates de tert-butyle (O) tel que décrits par Gipstein, J.Org.Chem, 1980, p1486 et les dérivés 1,1-bis-(methylsulfonyl)ethylene (P), 1-acetyl-1-methyl sulfonyl ethylene (Q), α-(methylsulfonyl) vinyl sulfonate de methyle (R), α-methylsulfonylacrylonitrile (S) tel que décrits par Shearer, US patent US2748050 : 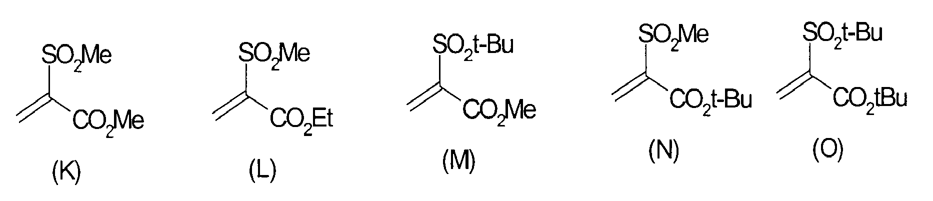 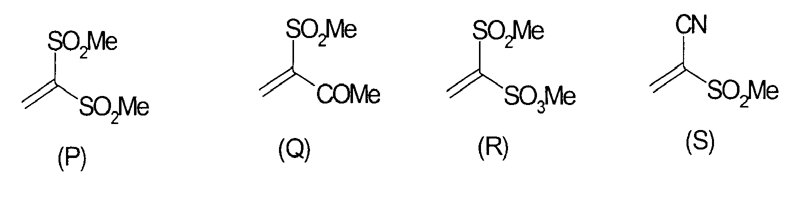
(v) les dérivés méthyl vinyl sulfone (T) et phényl vinyl sulfone (U) tel que décrits par Boor , J.Polymer Science, 1971, p249 : 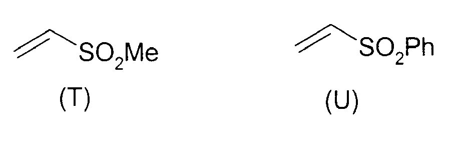
(vi) le dérivé phényl vinyl sulfoxide (V) tel que décrit par Kanga, Polymer preprints (ACS, Divison of Polymer Chemistry), 1987, p322 : 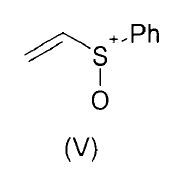
(vii) le dérivé 3-methyl-N-(phenylsulfonyl)-1-aza-1,3-butadiene (W) tel que décrit par Bonner, Polymer Bulletin, 1992, p517 : 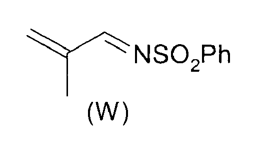
(viii) les dérivés acrylates et acrylamides comme :
   - le N-propyl-N-(3-triisopropoxysilylpropyl)acrylamide (X) et N-propyl-N-(3-triethoxysilylpropyl)acrylamide (Y) tel que décrit par Kobayashi, Journal of Polymer Science, Part A: Polymer Chemistry, 2005, p2754 : 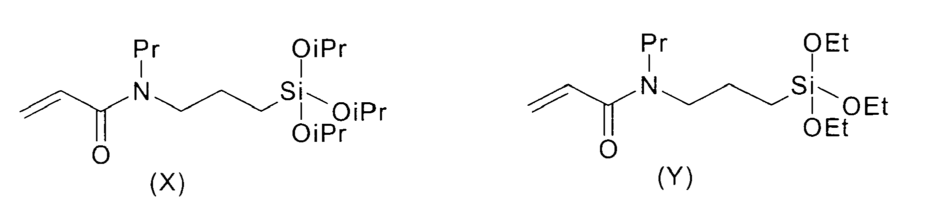
   - le 2-hydroxyethyl acrylate (Z) et le 2-hydroxyethyl méthacrylate (AA) tel que décrits par Rozenberg, International Journal of Plastics Technology, 2003, p17 : 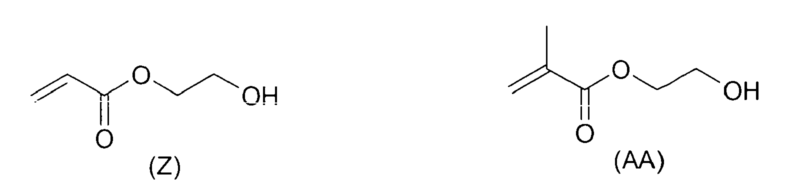
   - le n-butyl acrylate (AB) tel que décrit par Schmitt, Macromolecules, 2001, p2115, et le tert-butyl acrylate (AC) tel que décrit par Ishizone, Macromolecules, 1999, p955 :

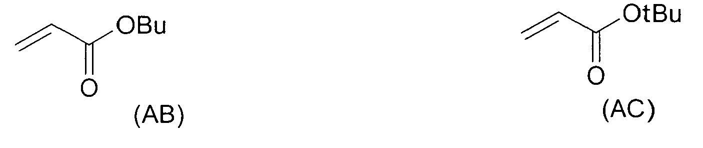

Le monomère électrophile, ou électro-attracteur, utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères électrophiles présentant au moins deux groupes électro-attracteurs, on peut citer les monomères de formule (1) : 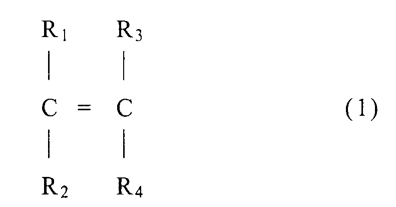 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène,

R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phényloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène et un résidu de polymère pouvant être obtenu par polymérisation radicalaire ou par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou -(CH₂)ₙ-(CF₂)ₘ-CHF₂ avec n=1 à 20 et m= 1 à 20.

Les substituants R₁ à R₄ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (2) : 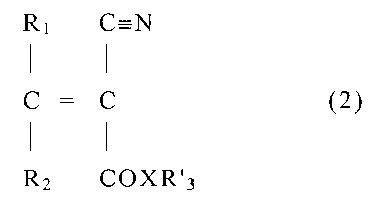 X désignant NH,S,O,
R₁ et R₂ ayant les mêmes significations que précédemment,
R'₃ pouvant désigner un atome d'hydrogène ou un groupe R tel que défini pour la formule (1).

De préférence, X désigne O.

A titre de composés de formule (2), on peut citer les monomères :
a) appartenant à la famille des 2-cyanoacrylates de polyfluoroalkyle en C₁₋₂₀ tels que :
   l'ester 2,2,3,3-tétrafluoropropylique de l'acide 2-cyano-2-propénoïque de formule : 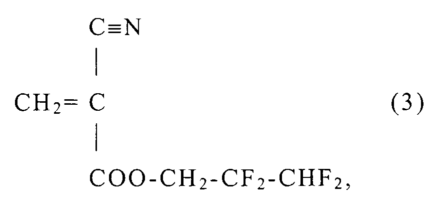
   ou encore l'ester 2,2,2-trifluoroéthylique de l'acide 2-cyano-2-propénoïque de formule : 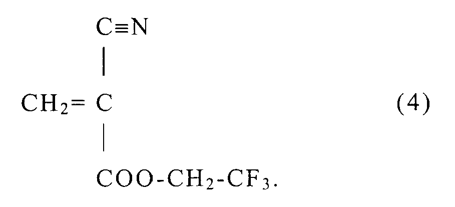
b) les cyanoacrylates d'alkyle en C₁-C₁₀ ou d'(alcoxy en C₁₋C₄)(alkyle en C₁-C₁₀).
   On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères le plus particulièrement préférés sont ceux de formule (5) et leurs mélanges : 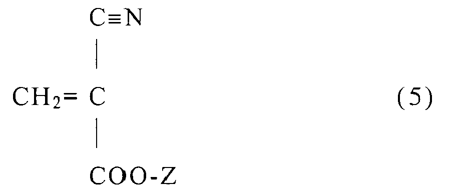 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Par milieu cosmétiquement acceptable, on entend un milieu compatible avec les matières kératiniques telles que les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique et l'alcool béhénylique, les acides gras en C₁₀-C₃₀ tels que l'acide laurique et l'acide stéarique, les amides gras en C₁₀-C₃₀ tels que le diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 10⁵ Pa (760mm de Hg).

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, non ioniques ou amphotères, des polymères fixants ou non, des polyols, des protéines, des vitamines, des colorants directs ou d'oxydation, des agents nacrants, des propulseurs, et des épaississants minéraux ou organiques tels que le benzylidène-sorbitol et les N-acylaminoacides.

Ces agents peuvent être éventuellement encapsulés. La capsule peut être de type polycyanoacrylate.

Le propulseur est notamment utilisé pour la préparation de compositions aérosol. Il comprend généralement des gaz comprimés et/ou liquéfiés bien connus dans la technique. De préférence, on utilise l'air, le gaz carbonique, l'azote comprimé ou encore un gaz comme le diméthyléther, les hydrocarbures halogénés, par exemple fluorés, ou non, ou leur mélange.

La composition est utilisée sur les matières kératiniques, et plus particulièrement sur les fibres kératiniques comme les cheveux, de préférence en présence d'un agent nucléophile, pour leur traitement cosmétique.

Le procédé de traitement cosmétique selon l'invention comprend l'application d'une composition telle que définie ci-dessus, sur les matières kératiniques, et plus particulièrement sur les fibres kératiniques comme les cheveux, en présence d'un agent nucléophile tel que défini ci-dessous.

Les agents nucléophiles susceptibles d'initier la polymérisation anionique sont des systèmes connus en eux-mêmes, capables de générer un carbanion au contact d'un monomère électrophile. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

Les agents nucléophiles particulièrement préférés sont les ions hydroxyle, notamment ceux présents dans l'eau. Cette eau peut être apportée par une humidification préalable des matières kératiniques.

Il est également possible, afin de moduler la cinétique de réaction, de préalablement humidifier les matières kératiniques, et notamment les fibres kératiniques comme les cheveux, à l'aide d'une solution aqueuse dont le pH a été ajusté à l'aide d'une base, d'un acide ou d'un mélange acide/base. L'acide et/ou la base peuvent être inorganiques ou organiques.

Selon un mode préféré de réalisation, la composition comprenant le monomère électrophile est exempte d'agent nucléophile.

Selon un autre mode préféré de réalisation, l'agent nucléophile est apporté par une seconde composition, appliquée sur ou sous le dépôt formé par l'application de la composition comprenant le monomère électrophile.

Il est également possible de moduler la cinétique de polymérisation par voie anionique en pré-imprégnant les matières kératiniques, et notamment les fibres kératiniques comme les cheveux, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des matières kératiniques, et notamment des fibres kératiniques comme les cheveux, par transformation chimique de la matière kératinique.

A titre d'exemple de transformation chimique, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions telles que décrites ci-dessus peut être suivie ou non d'un rinçage.

Elles peuvent se présenter sous la forme de lotion, de spray, de mousse, et être appliquées comme shampoing ou après-shampoing.

Un autre objet de l'invention consiste en un kit comprenant une première composition contenant au moins un monomère électrophile tel que défini ci-dessus et éventuellement au moins un inhibiteur de polymérisation anionique et/ou radicalaire tels que définis ci-dessus, ainsi qu'une deuxième composition comprenant dans un milieu cosmétiquement acceptable au moins un sel organique non polymérique présentant un point de fusion inférieur à 60 °C tel que défini ci-dessus.

L'invention a encore pour objet un procédé de traitement cosmétique des matières kératiniques, comprenant au moins deux étapes, une étape comprenant l'application du sel organique non polymérique présentant un point de fusion inférieur à 60 °C tel que défini ci-dessus, et une autre étape comprenant l'application d'au moins un monomère électrophile tel que défini ci-dessus, l'ordre des étapes étant indifférent.

Un mode de réalisation particulier de l'invention consiste en ce que l'application de l'acide organique non-réducteur se fait avant l'application d'au moins un monomère électrophile.

Les exemples suivants sont donnés à titre illustratif de la présente invention.

Dans les exemples suivants, toutes les quantités sont indiquées en pour cent en poids de matière active par rapport au poids total de la composition, sauf indication contraire.

### EXEMPLES

### Exemples de composition rincée

On a préparé un shampoing selon l'invention (exemple 1) et trois shampoings comparatifs (exemples comparatifs 1, 2 et 3) à partir des ingrédients suivants :

| | Ex. 1 | Ex. comp. 1 | Ex. comp. 2 | Ex. comp. 3 |
|---|---|---|---|---|
| Laurethsulfate de sodium | 30% | 30% | 30% | 30% |
| Cocobétaïne | 4% | 4% | 4% | 4% |
| Cocamide monoisopropanolamine | 2% | 2% | 2% | 2% |
| Tétrafluoroborate de 1-méthyl-3-hexylimidazolium | 2% | - | 1,5% | - |
| 2-cyanoacrylate de n-octyle | 1,5% | - | - | 5% |
| Polydiméthylsiloxane (250 000 cSt) | - | 1,5% | - | - |
| Cétéarylsulfate de sodium | 0,8% | 0,8% | 0,8% | 0,8% |
| POLYQUATERNIUM-10 | 0,4% | 0,4% | 0,4% | 0,4% |
| CARBOMER 980 | 0,2% | 0,2% | 0,2% | 0,2% |
| Propylèneglycol | 0,1% | 0,1% | 0,1% | 0,1% |
| Conservateur | qs | qs | qs | qs |
| Parfum | qs | qs | qs | qs |
| Eau qsp | 100 | 100 | 100 | 100 |

### Mode d'application 1

2g de chacune des compositions des exemple 1, exemples comparatifs 1 à 3, sont appliqués sur des mèches constituées de 2,7g de cheveux sensibilisés. Après deux minutes de contact des compositions avec les mèches de cheveux, celles-ci sont rincées, puis séchées 1 heure à température ambiante (environ 20-25 °C).

### Mode d'application 2

Des mèches constituées de 2,7g de cheveux sensibilisés sont humidifiées à l'aide de 1 ml d'eau par mèche. 2g de chacune des compositions des exemple 1, exemples comparatifs 1 à 3, sont appliqués sur ces mèches humidifiées. Après deux minutes de contact des compositions avec les mèches de cheveux, celles-ci sont rincées, puis séchées 1 heure à température ambiante (environ 20-25 °C).

Pour chaque mèche, le toucher et la brillance des cheveux sont évalués par un panel de 10 personnes. Une mèche vierge de même nature est utilisée comme référence.

L'évaluation tactile et visuelle des diverses mèches de cheveux est répétée avec la même procédure après avoir réalisé successivement 5 shampooings commercialisés sous la dénomination DOP camomille.

Les résultats d'évaluation sensorielle sont indiqués dans les tableaux suivants :

| Mode d'application 1 | | | | |
|---|---|---|---|---|
| Nature du traitement | Ex. 1 | Ex. comp. 1 | Ex. comp. 2 | Ex. comp. 3 |
| Après application | Douceur 3 Brillance 4 | Douceur 2 Brillance 1 | Douceur 2 Brillance 4 | Douceur 3 Brillance 1 |
| Après 5 shampoings | Douceur 2 Brillance 3 | Douceur 0 Brillance 0 | Douceur 0 Brillance 1 | Douceur 2 Brillance 1 |

| Mode d'application 2 | | | | |
|---|---|---|---|---|
| Nature du traitement | Ex. 1 | Ex. comp. 1 | Ex. comp. 2 | Ex. comp. 3 |
| Après application | Douceur 4 Brillance 4 | Douceur 2 Brillance 1 | Douceur 2 Brillance 4 | Douceur 4 Brillance 1 |
| Après 5 shampoings | Douceur 3 Brillance 3 | Douceur 0 Brillance 0 | Douceur 0 Brillance 1 | Douceur 3 Brillance 1 |

| | | | | |
|---|---|---|---|---|
| La notation est : | | | | |
| 0 = équivalent à la mèche non traitée, | | | | |
| 5 = très supérieur à la mèche non traitée. | | | | |

La douceur et la brillance apportées par la composition selon l'invention sont supérieures ou proches des compositions des exemples comparatifs juste après l'application de la composition.

Après 5 shampoings, l'apport de douceur et de brillance est mieux conservé lorsque les mèches sont traitées avec la composition selon l'invention, de l'exemple 1.

### Exemples de composition non rincée

On a préparé une crème de brillance selon l'invention (exemple 2) et trois crèmes de brillance comparatives (exemples comparatifs 4, 5 et 6) à partir des ingrédients suivants :

| | Ex. 2 | Ex. Comp. 4 | Ex. Comp. 5 | Ex. Comp. 6 |
|---|---|---|---|---|
| Tétrafluoroborate de 1-méthyl-3-hexylimidazolium | 1,5% | - | 1,5% | - |
| 2-cyanoacrylate de N-octyle | 5% | - | - | 5% |
| Cyclopentasiloxane | 10% | 10% | 10% | 10% |
| Cyclopentasiloxane dimethicone copolyol | 0,5% | 0,5% | 0,5% | 0,5% |
| Propylèneglycol | 2,5% | 2,5% | 2,5% | 2,5% |
| conservateur | qs | qs | qs | qs |
| parfum | qs | qs | qs | qs |
| Eau qsp | 100 | 100 | 100 | 100 |

### Mode d'application 1

2g de chacune des compositions des exemple 2, exemples comparatifs 4 à 6, sont appliqués sur des mèches constituées de 2,7g de cheveux sensibilisés. Après application, les mèches de cheveux sont séchées au casque 30 minutes à 70°C.

### Mode d'application 2

Des mèches constituées de 2,7g de cheveux sensibilisés sont humidifiées à l'aide de 1ml d'eau par mèche. 2g de chacune des compositions des exemple 2, exemples comparatifs 4 à 6, sont appliqués sur ces mèches humidifiées. Après application, les mèches de cheveux sont séchées au casque 30 minutes à 70°C.

Pour chaque mèche, le toucher et la brillance des cheveux sont évalués par un panel de 10 personnes. Une mèche vierge de même nature est utilisée comme référence.

L'évaluation tactile et visuelle des diverses mèches de cheveux est répétée avec la même procédure après avoir réalisé successivement 5 shampooings commercialisés sous la dénomination DOP camomille.

Les résultats d'évaluation sensorielle sont indiqués dans les tableaux suivants :

| Mode d'application 1 | | | | |
|---|---|---|---|---|
| Nature du traitement | Ex. 2 | Ex. comp. 4 | Ex. comp. 5 | Ex. comp. 6 |
| Après application | Douceur 4 Brillance 5 | Douceur 3 Brillance 3 | Douceur 3 Brillance 5 | Douceur 4 Brillance 2 |
| Après 5 shampoings | Douceur 3 Brillance 4 | Douceur 0 Brillance 0 | Douceur 0 Brillance 1 | Douceur 3 Brillance 1 |

| Mode d'application 2 | | | | |
|---|---|---|---|---|
| Nature du traitement | Ex. 2 | Ex. comp. 4 | Ex. comp. 5 | Ex. comp. 6 |
| Après application | Douceur 5 Brillance 5 | Douceur 3 Brillance 3 | Douceur 3 Brillance 5 | Douceur 5 Brillance 2 |
| Après 5 shampoings | Douceur 4 Brillance 4 | Douceur 0 Brillance 0 | Douceur 0 Brillance 1 | Douceur 4 Brillance 1 |

| | | | | |
|---|---|---|---|---|
| La notation est : | | | | |
| 0 = équivalent à la mèche non traitée, | | | | |
| 5 = très supérieur à la mèche non traitée. | | | | |

La douceur et la brillance apportées par la composition selon l'invention sont supérieures ou proches des compositions des exemples comparatifs juste après l'application de la composition.

Après 5 shampoings, l'apport de douceur et de brillance est mieux conservé lorsque les mèches sont traitées avec la composition selon l'invention, de l'exemple 2.

### Autres exemples de compositions non rincées

### Exemple 3 :

| | Ex 3 | Comparatif |
|---|---|---|
| Tétrafluoroborate de 1-méthyl-3-hexylimidazolium | 1,5% | - |
| Methylheptylcyanoacrylate* | 5% | 5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * commercialisé par la Société Chemence | | |

### Exemple 4 :

| | Ex 4 | Comparatif |
|---|---|---|
| Tétrafluoroborate de 1-méthyl-3-hexylimidazolium | 1,5% | - |
| Methoxyethylcyanoacrylate* | 5% | 5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * MO 460 commercialisé par la Société Tong Shen | | |

### Exemple 5 :

| | Ex 5 | Comparatif |
|---|---|---|
| Tétrafluoroborate de 1-méthyl-3-hexylimidazolium | 1,5% | - |
| Ethoxyethylcyanoacrylate* | 5% | 5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * EO-460 commercialisé par la Société Tong Shen | | |

### Exemple 6 :

| | Ex 6 | Comparatif |
|---|---|---|
| Tétrafluoroborate de 1-méthyl-3-hexylimidazolium | 1,5% | - |
| Butylcyanoacrylate* | 5% | 5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * B-60 commercialisé par la Société Tong Shen | | |

### Exemple 7 :

| | Ex 7 | Comparatif |
|---|---|---|
| Tétrafluoroborate de 1-méthyl-3-hexylimidazolium | 1,5% | - |
| Ethylcyanoacrylate* | 5% | 5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * Cyanolit 202 commercialisé par la Société Loctite | | |

### Exemple 8 :

| | Ex 8 | Comparatif |
|---|---|---|
| Tétrafluoroborate de 1-méthyl-3-hexylimidazolium | 1,5% | - |
| Ethylhexylcyanoacrylate* | 5% | 5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * O-60 commercialisé par la Société Tong Shen | | |

### Exemple 9 :

| | Ex 9 | Comparatif |
|---|---|---|
| Tétrafluoroborate de 1-méthyl-3-hexylimidazolium | 1,5% | - |
| n-octylcyanoacrylate* | 5% | 5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * commercialisé par la Société Chemence | | |

### Exemple 10 :

| | Ex 10 | Comparatif |
|---|---|---|
| Tétrafluoroborate de 1-méthyl-3-hexylimidazolium | 1,5% | - |
| Methylheptylcyanoacrylate* | 4.5% | 4.5% |
| Ethylhexylcyanoacrylate** | 0.5% | 0.5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * commercialisé par la Société Chemence | | |
| ** O-60 commercialisé par la Société Tong Shen | | |

### Exemple 11 :

| | Ex 1 | Comparatif |
|---|---|---|
| Tétrafluoroborate de 1-méthyl-3-hexylimidazolium | 1,5% | - |
| Methylheptylcyanoacrylate* | 3.5% | 3.5% |
| Butylcyanoacrylate** | 1.5% | 1.5% |
| Cyclopentasiloxane | 10% | 10% |
| Cyclopentasiloxane dimethicone copolyol | 0,5% | 0,5% |
| Propylèneglycol | 2,5% | 2,5% |
| conservateur | qs | qs |
| parfum | qs | qs |
| Eau qsp | 100 | 100 |

| | | |
|---|---|---|
| * commercialisé par la Société Chemence | | |
| ** B-60 commercialisé par la Société Tong Shen | | |

## Revendications

1. Composition comprenant dans un milieu cosmétiquement acceptable, au moins un monomère électrophile, et au moins un sel organique non polymérique présentant un point de fusion inférieur à 60°C.

2. Composition selon la revendication 1, **caractérisée en ce que** le sel organique présente un point de fusion inférieur à 20 °C, de préférence inférieur à 0 °C.

3. Composition selon la revendication 1 ou 2, **caractérisée en ce que** le sel organique est choisi parmi les sels d'imidazolium, de pyrazolium, de pyridinium, de pyrimidinium, de tétra-alkylphosphonium et de tétra-alkylammonium, et leurs mélanges.

4. Composition selon la revendication 3, **caractérisée en ce que** les sels d'imidazolium répondent à la formule suivante : 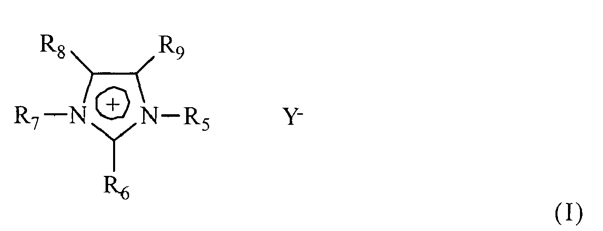 dans laquelle R₅ et R₇, identiques ou différents, représentent chacun un groupe alkyle comportant de 1 à 12 atomes de carbone,
R₆, R₈ et R₉, identiques ou différents, représentent chacun un atome d'hydrogène ou un groupe alkyle comportant de 1 à 4 atomes de carbone, et
Y⁻ représente un anion.

5. Composition selon la revendication 3, **caractérisée en ce que** les sels de pyrazolium répondent à la formule suivante : 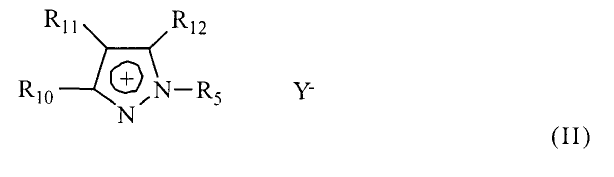 dans laquelle R₅ représente un groupe alkyle comportant de 1 à 12 atomes de carbone,
R₁₀, R₁₁ et R₁₂, identiques ou différents, représentent chacun un atome d'hydrogène ou un groupe alkyle comportant de 1 à 5 atomes de carbone, et
Y⁻ représente un anion.

6. Composition selon la revendication 3, **caractérisée en ce que** les sels de pyridinium répondent à la formule suivante : 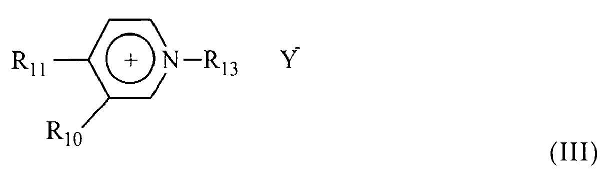 dans laquelle R₁₃ représente un groupe alkyle comportant de 1 à 12 atomes de carbone,
R₁₀ et R₁₁, identiques ou différents, représentent chacun un atome d'hydrogène ou un groupe alkyle comportant de 1 à 5 atomes de carbone, et
Y⁻ représente un anion.

7. Composition selon la revendication 3, **caractérisée en ce que** les sels de pyrimidinium répondent aux formules suivantes : 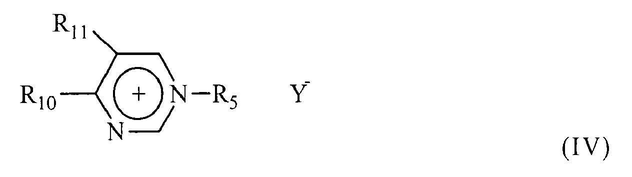 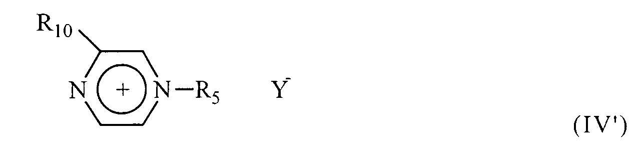 dans lesquelles R₅ représente un groupe alkyle comportant de 1 à 12 atomes de carbone,
R₁₀ et R₁₁, identiques ou différents, représentent chacun un atome d'hydrogène ou un groupe alkyle comportant de 1 à 5 atomes de carbone, et
Y⁻ représente un anion.

8. Composition selon la revendication 3, **caractérisée en ce que** les sels de tétra-alkylphosphonium répondent à la formule suivante : 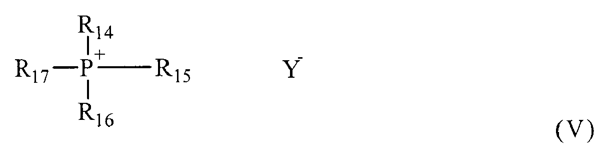 dans laquelle R₁₄, R₁₅, R₁₆ et R₁₇, identiques ou différents, représentent un groupe alkyle comportant de 1 à 18 atomes de carbone, et
Y⁻ représente un anion.

9. Composition selon la revendication 3, **caractérisée en ce que** les sels de tétra-alkylammonium répondent à la formule suivante : 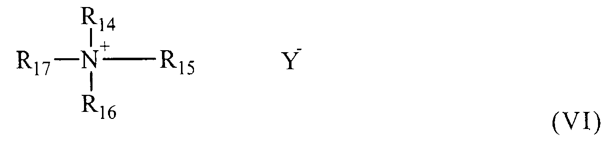 dans laquelle R₁₄, R₁₅, R₁₆ et R₁₇, identiques ou différents, représentent un groupe alkyle comportant de 1 à 18 atomes de carbone, et
Y⁻ représente un anion.

10. Composition selon l'une quelconque des revendications 4 à 9, **caractérisée en ce que** les anions sont choisis parmi les ions chlorure (Cl⁻), bromure (Br⁻), tétrachloroaluminate (AlCl₄⁻), tétrachloronickel (NiCl₄⁻), perchlorate (ClO₄⁻), nitrate (NO₃⁻), nitrite (NO₂⁻), sulfate (SO₄²⁻), méthylsulfate (CH₃SO₄⁻), tétrafluoroborate (BF₄⁻), hexafluorophosphate (PF₆⁻), hexafluoroantimonate (SbF₆⁻), triflate [TfO] (CF₃SO₂⁻), nonaflate [NfO] (CF₃(CF₂)₃SO₂⁻), un ion [Tf₂N] (CF₃SO₂)₂N⁻), trifluoroacétate [TA] (CF₃CO₂⁻), heptafluorobutanoate [HB] (CF₃(CF₂)₃CO₂⁻), acétate (CH₃CO₂⁻) et trifluorométhanesulfonate (CF₃SO₃⁻).

11. Composition selon l'une quelconque des revendications précédentes, **caractérisée en ce que** le sel organique est choisi parmi :
le chlorure de 1-éthyl-3-méthylimidazolium,
le bromure de 1-éthyl-3-méthylimidazolium,
le chlorure de 1-butyl-3-méthylimidazolium,
le chlorure de 1-hexyl-3-méthylimidazolium,
le chlorure de 1-méthyl-3-octylimidazolium,
le chlorure de 1-décyl-3-méthylimidazolium,
le bromure de 1-décyl-3-méthylimidazolium,
le chlorure de 1-dodécyl-3-méthylimidazolium,
le chlorure de 1-méthyl-3-tétradécylimidazolium,
le chlorure de 4-méthyl-N-butyl-pyridinium,
le chlorure de 3-méthyl-N-butylpyridinium,
le chlorure de 4-méthyl-N-hexylpyridinium,
le tétrafluoroborate de 1-éthyl-3-méthylimidazolium,
le tétrafluoroborate de 1-butyl-3-méthylimidazolium,
le tétrafluoroborate de 1-pentyl-3-méthylimidazolium,
le tétrafluoroborate de 1-hexyl-3-méthylimidazolium,
le tétrafluoroborate de 1-heptyl-3-méthylimidazolium,
le tétrafluoroborate de 1-octyl-3-méthylimidazolium,
le tétrafluoroborate de 1-nonyl-3-méthylimidazolium,
le tétrafluoroborate de 1-décyl-3-méthylimidazolium,
le tétrafluoroborate de 4-méthyl-N-butylpyridinium,
le tétrafluoroborate de 1-hexyl-3-éthylimidazolium,
l'hexafluorophosphate de 1-éthyl-3-méthylimidazolium,
l'hexafluorophosphate de 1-butyl-3-méthylimidazolium,
l'hexafluorophosphate de 1-pentyl-3-méthylimidazolium,
l'hexafluorophosphate de 1-hexyl-3-méthylimidazolium,
l'hexafluorophosphate de 1-heptyl-3-méthylimidazolium,
l'hexafluorophosphate de 1-octyl-3-méthylimidazolium,
l'hexafluorophosphate de 1-nonyl-3-méthylimidazolium,
l'hexafluorophosphate de 1-décyl-3-méthylimidazolium,
le méthylsulfate de 1,3-diméthylimidazolium,
le méthylsulfate de 1-méthyl-3-butylimidazolium,
le nitrate de 1-éthyl-3-méthylimidazolium,
le nitrite de 1-éthyl-3-méthylimidazolium,
l'acétate de 1-éthyl-3-méthylimidazolium,
le sulfate de 1-éthyl-3-méthylimidazolium,
les triflates de 1-éthyl-3méthylimidazolium,
les nonaflates de 1-éthyl-3-méthylimidazolium,
le bis(trifyl)amide de 1-éthyl-3-méthylimidazolium,
le bromure de 1-butylpyridinium,
le trifluorométhanesulfonate de 1-butylpyrimidinium,
le trifluorométhanesulfonate de 1-hexylpyrimidinium,
le trifluoroacétate de 1-éthyl-3-méthylimidazolium,
le chlorure de trihexyl-tétradécyl-phosphonium,
le chlorure de tributyl-tétradécyl-phosphonium,
le tétrafluoroborate de 1-éthyl-2-méthylpyrazolium,
le tétrafluoroborate de 1-méthyl-3-butylpyrimidinium,
le trifluoroacétate de 1-éthyl-3-méthylimidazolium, et
le sulfate de trioctylammonium.

12. Composition selon l'une quelconque des revendications précédentes, **caractérisée en ce que** le sel organique est contenu en une quantité comprise entre 0,001% et 95% en poids, de préférence entre 0,01% et 50% en poids par rapport au poids total de la composition.

13. Composition selon l'une quelconque des revendications précédentes, **caractérisée en ce que** le ou les monomères électrophiles répondent à la formule : 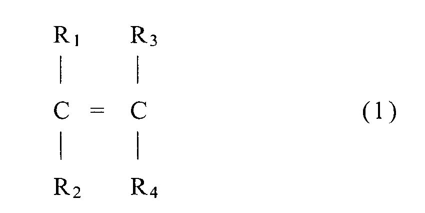 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR, -COOR, -COR,
- SH, -SR, -OH et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, -S(R)₂⁺, -SH₂⁺, -NH₃⁺, -NO₂ -SO₂R -C≡N, -COOH, -COOR, -COSR, -CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle et aryloxy.
R désigne un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR', -COOR', -COR', -SH, -SR', -OH, les atomes d'halogène, et un résidu de polymère pouvant être obtenu par polymérisation radicalaire ou par polycondensation ou par ouverture de cycle, R' désignant un groupe alkyle en C₁-C₁₀.

14. Composition selon la revendication 13, **caractérisée en ce que** le ou les monomères électrophiles sont choisis parmi les composés de formule : 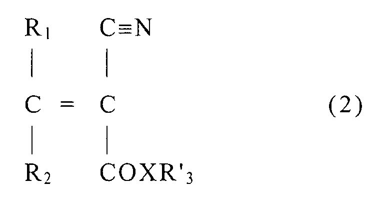 X désignant NH,S,O,
R₁ et R₂ sont tels que définis dans la revendication 13,
R'₃ pouvant désigner un atome d'hydrogène ou un groupe R tel que défini dans la revendication 13.

15. Composition selon la revendication 14, **caractérisée par le fait que** le ou les monomères sont choisis parmi les 2-cyanoacrylates de polyfluoroalkyle en C₁₋₂₀, les cyanoacrylates d'alkyle en (C₁-C₁₀) ou d'(alcoxy en C₁-C₄)(alkyle en C₁-C₁₀).

16. Composition selon la revendication 15, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

17. Composition selon la revendication 16, **caractérisée en ce que** le ou les monomères électrophiles répondent à la formule (5) : 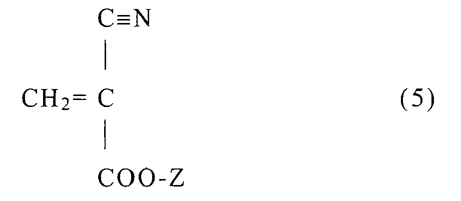 dans laquelle : Z=-(CH₂)₇-CH₃,
- CH(CH₃)-(CH₂)₅-CH₃,
- CH₂-CH(C₂H₅)-(CH₂)₃-CH₃,
- (CH₂)₅-CH(CH₃)-CH₃,
- (CH₂)₄-CH(C₂H₅)-CH₃.

18. Composition selon l'une quelconque des revendications précédentes, **caractérisée en ce que** le ou les monomères électrophiles sont fixés de façon covalente sur des supports tels que des polymères, des oligomères ou des dendrimères.

19. Composition selon l'une quelconque des revendications précédentes, **caractérisée en ce que** la quantité de monomère électrophile va de 0,001 à 80 % en poids, de préférence de 0,1 à 40 % en poids par rapport au poids total de la composition.

20. Composition selon l'une quelconque des revendications précédentes, **caractérisée en ce que** le milieu cosmétiquement acceptable est anhydre.

21. Composition selon la revendication 20, **caractérisée en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

22. Composition selon l'une quelconque des revendications précédentes, **caractérisée en ce que** la composition contient des inhibiteurs de polymérisation.

23. Composition selon la revendication 22, **caractérisée en ce que** les inhibiteurs sont des inhibiteurs de polymérisation anioniques et/ou radicalaires.

24. Composition selon la revendication 22 ou 23, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges.

25. Composition selon l'une quelconque des revendications 22 à 24, **caractérisée en ce que** les inhibiteurs de polymérisation sont présents en des quantités allant de 10 ppm à 20 % par rapport au poids total de la composition.

26. Composition selon l'une quelconque des revendications précédentes, **caractérisée en ce que** la composition contient en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, non ioniques ou amphotères, les polymères fixants ou non, les polyols, les protéines, des vitamines, les colorants directs ou d'oxydation, et les agents nacrants.

27. Composition selon la revendication 26, **caractérisée en ce que** l'agent est encapsulé.

28. Utilisation de la composition selon l'une quelconque des revendications précédentes, pour le traitement cosmétique des matières kératiniques.

29. Utilisation selon la revendication 28, pour le traitement cosmétique des fibres kératiniques, et en particulier des cheveux.

30. Utilisation selon la revendication 28 ou 29, en présence d'un agent nucléophile.

31. Utilisation selon la revendication 30, **caractérisée en ce que** l'agent nucléophile est choisi parmi les composés moléculaires, les oligomères, les dendrimères ou polymères possédant des fonctions nucléophiles choisies parmi : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

32. Utilisation selon la revendication 31, **caractérisée en ce que** l'agent nucléophile est de l'eau.

33. Procédé de traitement cosmétique des matières kératiniques, comprenant l'application d'une composition selon l'une quelconque des revendications précédentes 1 à 27, sur les matières kératiniques en présence d'un agent nucléophile.

34. Procédé selon la revendication 33, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃―, ClO₄⁻ et H₂O, Ph représentant le groupe phényle, Ar représentant un groupe aryle et R représentant un groupe alkyle en C₁-C₁₀.

35. Procédé selon la revendication 34, **caractérisé en ce que** l'agent nucléophile est l'eau.

36. Procédé selon l'une quelconque des revendications 33 à 35, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

37. Procédé selon l'une quelconque des revendications 33 à 35, **caractérisé en ce que** les matières kératiniques sont pré-imprégnées à l'aide d'un agent nucléophile différent de l'eau.

38. Procédé selon l'une quelconque des revendications 33 à 35, **caractérisé en ce que** les matières kératiniques sont préalablement réduites avant application de la composition.

39. Procédé selon l'une des revendications 33 à 38, **caractérisé en ce que** l'application de la composition est suivie d'un rinçage.

40. Procédé selon l'une des revendications 33 à 39, **caractérisé en ce que** les matières kératiniques sont les cheveux.

41. Kit comprenant une première composition contenant au moins un monomère électrophile tel que défini dans l'une quelconque des revendications 13 à 18, et éventuellement au moins un inhibiteur de polymérisation anionique et/ou radicalaire, ainsi qu'une deuxième composition comprenant dans un milieu cosmétiquement acceptable au moins un sel organique non polymérique présentant un point de fusion inférieur à 60 °C tel que défini dans l'une quelconque des revendications 2 à 11.

42. Procédé de traitement cosmétique des matières kératiniques, comprenant au moins deux étapes, une étape comprenant l'application d'un sel organique non polymérique présentant un point de fusion inférieur à 60 °C tel que défini dans l'une quelconque des revendications 2 à 11, et une autre étape comprenant l'application d'au moins un monomère électrophile tel que défini dans l'une quelconque des revendications 13 à 18.

43. Procédé selon la revendication 42, **caractérisé en ce que** l'application du sel organique non polymérique présentant un point de fusion inférieur à 60 °C se fait avant l'application d'au moins un monomère électrophile.
